(19) Europäisches Patentamt
European Patent Office
Office européen des brevets

(11) Veröffentlichungsnummer: **0 108 908**
**B1**

(12) **EUROPÄISCHE PATENTSCHRIFT**

(45) Veröffentlichungstag der Patentschrift: **04.02.87**

(51) Int. Cl.⁴: **C 07 D 249/18,** A 01 N 43/647

(21) Anmeldenummer: **83109880.1**

(22) Anmeldetag: **04.10.83**

(54) **Neue Benzotriazole, ihre Herstellung und ihre Verwendung als biozide Wirkstoffe.**

(30) Priorität: **20.10.82 DE 3238804**

(43) Veröffentlichungstag der Anmeldung: **23.05.84 Patentblatt 84/21**

(45) Bekanntmachung des Hinweises auf die Patenterteilung: **04.02.87 Patentblatt 87/6**

(84) Benannte Vertragsstaaten: **BE CH DE FR GB IT LI NL**

(56) Entgegenhaltungen: **DE-A-2 713 871** **US-A-4 240 822**

(73) Patentinhaber: **CELAMERCK GmbH & Co. KG, Binger Strasse 173, D-6507 Ingelheim am Rhein (DE)**

(72) Erfinder: **Sehring, Richard, Dr., Frankenstrasse 13, D-6507 Ingelheim (DE)**
Erfinder: **Raddatz, Erich, Dr., Carrera 1-0e No. 5-265, Cali (CO)**
Erfinder: **Drandarevski, Christo, Dr., Boehringerstrasse 8, D-6507 Ingelheim (DE)**

**EP 0 108 908 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die Erfindung betrifft neue Benzotriazole der Formel

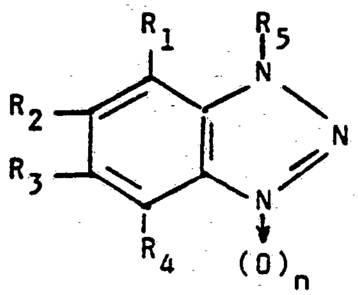

(I),

in der

n 0 oder 1,

$R_1$, $R_2$, $R_3$ und $R_4$ H,F, Chlor, Brom, $CF_3$, CN, $C_1$-$C_4$-Alkyl, OR oder SR bedeuten (R gleich gegebenenfalls sauerstoffunterbrochener und/oder chlorsubstituierter $C_1$-$C_6$-Alkylrest wobei H bis zu dreimal, CN, OR und SR maximal zweimal, $CF_3$ maximal einmal vorhanden sein kann,

$R_5$ einen $C_1$-$C_8$-Alkylrest oder $C_3$-$C_6$-Alkenyl darstellt,

mit der Maßgabe, daß im Falle n gleich 1 und $R_4$ gleich Wasserstoff die beiden Bedeutungskombinationen $R_1$ und $R_2$ Chlor, $R_3$ Wasserstoff sowie $R_1$, $R_2$ und $R_3$ Chlor ausgenommen werden,

die Herstellung dieser Verbindungen und ihre Verwendung als biozide Wirkstoffe.

In den obigen Definitionen können die Alkylreste innerhalb der verschiedenen Substituenten jeweils geradkettig oder verzweigt sein. Bevorzugte Alkylreste sind Methyl und Ethyl. Als sauerstoffunterbrochene Alkylreste sind z.B. zu nennen Methoxyethyl, n-Butoxyethyl, Methoxyisopropyl, t-Butoxy-ethyl.

Die Verbindungen der Formel 1 können nach den folgenden, z.T. überraschend verlaufenden Verfahren erhalten werden.

a) Man setzt ein Benzotriazol der Formel

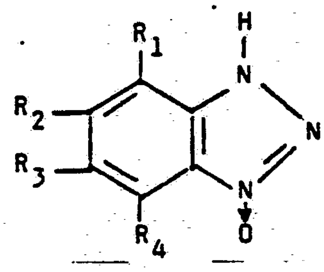

(IIa)

bzw. die tautomere Verbindung der Formel

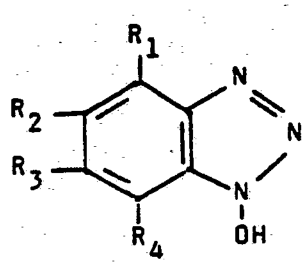

(IIb)

worin $R_1$ bis $R_4$ die obige Bedeutung haben mit einem für die Einführung des Restes $R_5$ geeigneten Schwefelsäure- bzw. Sulfonsäureester oder Bromid $R_5Br$, zweckmäßig in einem inerten Lösungsmittel bei erhöhter Temperatur um bei Verwendung von $R_5Br$ unter Zusatz einer katalytischen Menge eines Schwefelsäure-, eines Sulfonsaureesters oder eines üblichen Phasentransferkatalysators und führt gewünschtenfalls das erhaltene N-Oxid durch Umsetzung mit Phosphortrichlorid in die Verbindung mit n gleich 0 über.

Die intermediär entstehenden O-Alkyl- bzw. O-Allylverbindungen lagern sich unter den Reaktionsbedingungen in die Verbindungen der Formel I mit n gleich 1 um. Die Reduktion der N-Oxid-Gruppierung mit Phosphortrichlorid erfolgt gewünschtenfalls in an sich bekannter Weise.

Als Reaktionsmedium für die Einführung des Restes $R_5$ benutzt man inerte organische Lösungsmittel, wie Toluol, Benzol, Xylol, Chlorbenzol, und erhitzt mehrere Stunden lang auf Temperaturen zwischen etwa 80 und 150°C.

b) Man erhitzt eine Verbindung der Formel

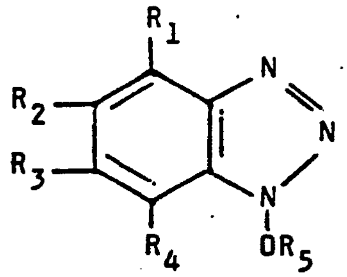

(III),

in der $R_1$ bis $R_5$ die obige Bedeutung haben, in Gegenwart eines geeigneten Schwefelsäure- oder Sulfonsäureesters oder einer geringen Menge eines üblichen Phasentransferkatalysators und reduziert gewünschtenfalls das resultierende N-Oxid mit $PCl_3$. -Bei diesem Verfahren kann auf ein Lösungsmittel verzichtet werden, da sich die Reaktion auch gut in der Schmelze durchführen läßt. Die Reaktionstemperatur liegt zwischen etwa 80 und 150°C. Im allgemeinen verläuft die Reaktion in kurzer Zeit. Als Katalysatoren auf der Reihe der Sulfonsäure- und Schwefelsäureester sind diejenigen geeignet, die auch zur Einführung des Restes $R_5$ bei Verfahren a) benutzt werden. Einfacher ist es im allgemeinen, Dimethylsulfat oder p-Toluolsulfonsäuremethylester als Katalysator zu verwenden.

Für Verfahren b) geeignete Phasentransferkatalysatoren sind etwa in der Monographie von E.V. Dehmlow und S.S. Dehmlow, "Phase Transfer Catalysis", Verlag Chemie, Weinheim (1980), insbesondere Seite 38 sowie Seite 39 - 43 zu entnehmen.

Zu nennen sind beispielsweise Triethylbenzylammoniumchlorid oder -bromid; Tetrabutylammoniumchlorid, -bromid, -hydrogensulfat, -hydroxid; Methyltrioctylammoniumchlorid; Tetrabutylphosphoniumbromid.

Die Katalysatormenge kann in weiten Grenzen variiert werden und zwischen ca. 1 : 10 und 1 : 100 liegen (Gewichtsverhältnis Katalysator/Ausgangsstoff). Insbesondere bei größeren Ansätzen kann die untere Grenze noch erheblich gesenkt werden.

c) Man setzt ein α-Halogennitrobenzol der Formel

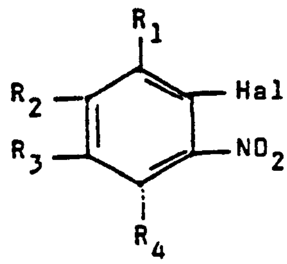

(IV),

worin $R_1$ bis $R_4$ die obige Bedeutung haben und Hal für Fluor, Chlor oder Brom steht, in einem inerten Lösungsmittel mit einem Hydrazin der Formel $R_5 - NH - NH_2$ (V), worin $R_5$ die obige Bedeutung hat, unter Zusatz von basischen Substanzen um und reduziert gewünschtenfalls das entstehende N-Oxid zu der entsprechenden Verbindung de Formel 1 mit n gleich O.

Die Umsetzung erfolgt in der Wärme, vorzugsweise bei der Siedetemperatur des Reaktionsgemischs. Als basische Verbindungen können insbesondere Alkalien, Alkalicarbonate oder starkbasische Amine verwendet werden.

d) Zur Herstellung erfindungsgemäßer Verbindungen, in denen n gleich 0 ist, kann ein entsprechendes Triazolderivat der Formel

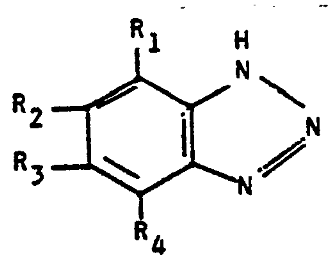

(VI),

worin $R_1$ bis $R_4$ die obige Bedeutung haben, in an sich bekannter Weise N-substituiert werden.

Dieses Verfahren eignet sich weniger zur Herstellung solcher Verbindungen, in denen zwei oder mehr der Reste $R_1$ bis $R_4$ Halogen bedeuten.

Zur Herstellung von Verbindungen mit einem höheren Substitutionsgrad ist gegebenenfalls die Anwendung der Verfahren a) oder b) günstiger.

Die Ausgangsstoffe für die erfindungsgemäßen Verfahren können, soweit sie noch nicht beschrieben sind, nach an sich bekannten Methoden gewonnen werden. So erhält man aus Verbindungen der obigen Formel IV mit Hydrazin in alkoholischer Lösung die Verbindungen der Formel IIa bzw. IIb, die durch Alkylierung bzw. Allylierung am Sauerstoff nach üblichen Verfahren die Verbindungen der Formel III ergeben.

Die Verbindungen der Formel VI können aus Phenylendiaminen der Formel

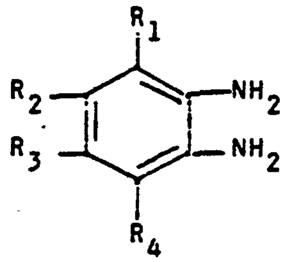

(VII),

worin $R_1$ bis $R_4$ die obige Bedeutung haben, durch Diazotieren und Ringschluß hergestellt werden.

Die Verbindungen der Formel I zeichnen sich durch eine starke fungizide Wirkung gegen phytopathogene Pilze aus, etwa gegen Rost- und Mehltaupilze. Hervorzuheben ist die Wirkung gegen wichtige Krankheitserreger in Reis, etwa Pellicularia, Helminthosporium und besonders Piricularia oryzae. Gegen diese Pilze sind weder die Ausgangsstoffe der Formeln IIa/IIb noch die der Formel III wirksam.

Für die Anwendung werden die erfindungsgemäßen Wirkstoffe zu üblichen Formulierungen verarbeitet, z.B. zu Suspensionspulvern, Stäuben, Granulaten, Emulsionen- bzw. Lösungskonzentraten.

Aus den Konzentraten werden Spritzbrühen mit im allgemeinen zwischen 0,01 und 0,1 Gewichtsprozent Wirkstoff durch Vermischen mit Wasser hergestellt. Die erfindungsgemäßen Wirkstoffe können in verschiedenartigen Kulturen eingesetzt werden, etwa in Reis, Getreide, Gurken-, Obst, Zierpflanzen.

Beispiele für die Formulierung:

**1. Emulsionskonzentrat**

5,0 Gew.-Teile Wirkstoff gemäß der Erfindung
3,4 Gew.-Teile epoxidiertes Pflanzenöl
13,4 Gew.-Teile eines Kombinationsemulgators
aus Fettalkoholpolyglykoläther
und Calcium-Alkylarylsulfonat
40,0 Gew.-Teile Dimethylformamid
38,2 Gew.-Teile Xylol

Die Komponenten werden vermischt und für die Anwendung mit Wasser auf eine Wirkstoffkonzentration von 0,01 bis 0,1 Gewichtsprozent verdünnt.

**2. Suspensionspulver**

10 Gew.-Teile Wirkstoff gemäß der Erfindung
3 Gew.-Teile Natrium Fettalkoholsulfonat
5 Gew.-Teile Salze von Naphthalin-sulfonsäure
Formaldehydkondensat
82 Gew.-Teile Kaolin

Verschiedene erfindungsgemäße Verbindungenwurden unter tropischen Bedingungen an Saatreis untersucht. Saatreis-Reihen zwischen älteren, iinfizierten Reihen wurden am 18., 23. und 29. Tag nach der Saat mit einer Spritzbrühe behandelt, die 500 ppm Wirkstoff enthielt. Als Vergleich diente die Wirkung eines üblichen Piricularia-Mittels sowie die nur mit Wasser behandelte Kontrolle.

Die erfindungsgemäßen Verbindungen erwiesen sich als stärker wirksam als die Vergleichsverbindung und als gut verträglich.

**Beispiel 1**

**3-Methyl-5-fluor-6-chlorbenzotriazol-1-N-oxid**

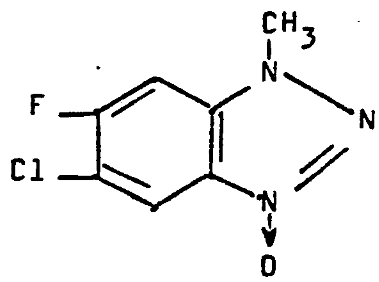

a) 10 g 5-Chlor-2,4-difluornitrobenzcl werden in 70 ml Ethanol gelöst und mit 5 g Natriumcarbonat versetzt. Zu dieser Mischung werden bei -5 bis 0°C 2,5 g Hydrazinhydrat, gelöst in 20 ml Ethanol, zugetropft. Das Reaktionsgemisch wird zum Rückfluß erhitzt. Das Lösungsmittel wird dann abdestilliert, der Rückstand in Wasser gelöst und mit 2 N Salzsäure angesäuert. Das Produkt wird aus Acetonitril umkristallisiert, Ausbeute 8 g eines dünnschichtchromatographisch einheitlichen Produkts, Fp. >260°C.

b) 6 g des Produkts werden mit 6,3 g Dimethylsulfat in 20 ml Toluol 1 Stunde unter Rückfluß gekocht. Nach dem Abkühlen wird mit Eiswasser versetzt und die ausgefallene Substanz abgesaugt. Nach Umkristallisation aus Propanol beträgt der Schmelzpunkt 238 - 240°C.

**Beispiel 2**

**3-Methyl-5-fluor-6-chlorbenzotriazol**

4,36 g des nach Beispiel 1b) erhaltenen N-Oxids werden in 20 ml Chloroform gelöst und mit 5 ml Phosphortrichlorid versetzt. Nach 3-stündigem Kochen unter Rückfluß wird das Lösungsmittel abdestilliert, erneut Chloroform zugegeben und mit Sodalösung neutralisiert. Man trocknet die organische Phase über Natriumsulfat, destilliert das Lösungsmittel ab und kristallisiert aus Cyclohexan um. Ausbeute 3,1 g, Fp. 93 - 94°C.

**Beispiel 3**

**3-Meth-yl-4-chlorbenzotriazol-1-N-oxid**

Aus 2,3-Dichlornitrobenzol wird analog Beispiel 1 a mit Hydrazinhydrat in Ethanol 4-Chlor-Benzotriazol-1-N-oxid hergestellt, Fp. 157 - 158°C.

5,1 g dieser Verbindung /0,02 mol) werden in 20 ml Toluol gelöst und mit 4 g Dimethylsulfat versetzt. Die Mischung wird 1 Stunde unter Rückfluß gekocht und nach dem Abkühlen mit Wasser gewaschen und getrocknet. Man destilliert das Lösungsmittel ab und destilliert den Rückstand aus Acetonitril um. Ausbeute 4,0 g, Fp. 196°C.

**Beispiel 4**

**3-Methyl-4-chlorbenzotriazol**

Analog Beispiel 2 wird durch Kochen der nach Beispiel 3 erhaltenen Verbindung mit Phosphortrichlorid in Chloroform die Titelverbindung erhalten.

Fp. 85°C (aus Cyclohexan). Ausbeute 95 % d.Th.

Analog werden die Beispiele der nachstehenden Tabelle erhalten:

**Tabelle**

Verbindungen der Formel

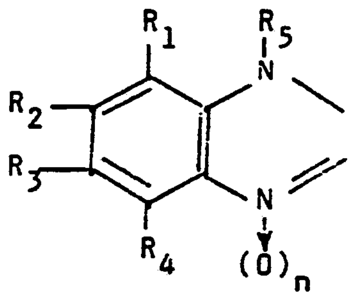

| Nr. | $R_1$ | $R_2$ | $R_3$ | $R_4$ | $R_5$ | n | phys.Konstanten |
|---|---|---|---|---|---|---|---|
| 1 | Cl | Br | H | H | $CH_2=CH-CH_2$ | 1 | Fp.105°C |
| 2 | Cl | F | H | H | $CH_3$ | 1 | Fp.139-144°C |
| 3 | Br | Cl | H | $OCH_3$ | $CH_3$ | 1 | Fp.175-180°C |
| 4 | H | H | CN | H | $CH_3$ | 1 | Fp. > 250°C |
| 5 | Cl | Cl | Cl | Cl | $CH_3$ | 1 | Fp.>250°C |
| 6 | Cl | Cl | H | H | $CH(CH_3)_2$ | 0 | Öl |
| 7 | H | H | H | Cl | $CH_3$ | 0 | Fp.70-72°C |
| 8 | H | Cl | Cl | H | $CH_3$ | 0 | Fp.196-200°C |
| 9 | H | H | Cl | Cl | $CH_3$ | 0 | Fp. 102 |
| 10 | Cl | Cl | H | H | $CH_3$ | 0 | Fp.99-100°C |
| 11 | Cl | Cl | H | H | $CH_2-CH_2Cl$ | 1 | Fp.85-90°C |
| 12 | Cl | Cl | H | H | $C_2H_5$ | 1 | Fp.130-132°C |
| 13 | CL | Cl | H | H | $n-C_7H_{15}$ | 1 | Öl |
| 14 | Cl | Cl | H | H | $n-C_7H_{15}$ | 0 | Öl |
| 15 | Cl | Cl | H | H | $n-C_4H_9$ | 1 | Fp.60-62°C |
| 16 | Cl | Cl | H | H | $n-C_4H_9$ | 0 | |
| 17 | H | Cl | Cl | H | $CH_2-CH_2Cl$ | 1 | Fp.172-174°C |
| 18 | Cl | $SC_2H_5$ | H | H | $CH_3$ | 1 | Öl |
| 19 | H | $SOC_2H_5$ | Cl | H | $CH_3$ | 1 | Fp.204°C |
| 20 | Cl | $CF_3$ | H | H | $CH_3$ | 1 | Fp.190-192°C |
| 21 | Cl | $CF_3$ | H | H | $CH_3$ | 0 | Öl |

**Patentansprüche**

1) Verbindungen der Formel

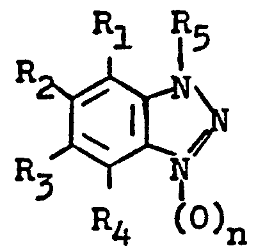

(I)

in der

n 0 oder 1,

$R_1$, $R_2$, $R_3$ und $R_4$, H, F, Chlor, Brom, $CF_3$, CN, $C_1$-$C_4$-Alkyl, OR oder SR bedeuten (R gleich gegebenenfalls sauerstoffunterbrochener und/oder chlorsubstituierter $C_1$-$C_6$-Alkylrest, wobei H bis zu dreimal, CN, OR und SR maximal zweimal, $CF_3$ maximal einmal vorhanden sein kann,

$R_5$ einen $C_1$-$C_8$-alkylrest oder $C_3$-$C_6$-alkenylrest darstellt,

mit der Maßgabe, daß im Falle n gleich 1 und $R_4$ gleich Wasserstoff die beiden Bedeutungskombinationen $R_1$ und $R_2$ Chlor, $R_3$ Wasserstoff sowie $R_1$, $R_2$ und $R_3$ Chlor ausgenommen werden.

2) Verbindungen nach Anspruch 1, worin n gleich 0 ist.

3) Verbindungen nach Anspruch 1, worin n gleich 1 ist.

4) Verbindungen nach Anspruch 1, 2 oder 3, worin $R_5$ Methyl ist.

5) 3-Methyl-4-chlor-benzotriazol

6) 3-Methyl-4,5-dichlor-benzotriazol 7) 3-Isopropyl-4,5-dichlor-benzotriazol

8) 3-Methyl-4-chlor-5-fluor-benzotriazol-N-oxid

9) 3-Methyl-5-fluor-6-chlor-benzotriazol-N-oxid

10) 3-Methyl-4,5,6,7-tetrachlor-benzotriazol-N-oxid

11) 3-Methyl-4-chlor-benzotriazol-N-oxid

12) Biozides Mittel, insbesondere zur Bekämpfung phytopathogener Pilze, gekennzeichnet durch einen Gehalt an einer Verbindung nach Anspruch 1 bis 11.

13) Verwendung von Verbindungen nach Anspruch 1 bis 11 bei der Bekämpfung phytopathogener Pilze.

14) Verfahren zur Herstellung von Verbindungen nach Anspruch 1 bis 11, dadurch gekennzeichnet, daß man

a) ein Benzotriazol der Formel

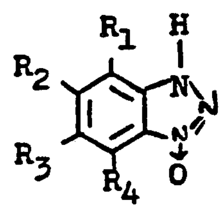

(IIa)

worin $R_1$ bis $R_4$ die obige Bedeutung haben, mit einem für die Einführung des Restes $R_5$ geeigneten Schwefelsäure- bzw. Sulfonsäureester oder mit einem Bromid $R_5$ Br umsetzt, wobei man im Falle der Reaktion mit $R_5$ Br in Gegenwart einer katalytischen Menge eines geeigneten Schwefelsäure- bzw. Sulfonsäureesters oder gegebenenfalls in Gegenwart eines üblichen Phasentransferkatalysators erhitzt und gewünschtenfalls die so erhaltenen Verbindungen der Formel I mit n = 1 in an sich bekannter Weise durch Umsetzung mit Phosphortrichlorid in Verbindungen der Formel 1 mit n = 0 überführt oder daß man

b) eine Verbindung der Formel

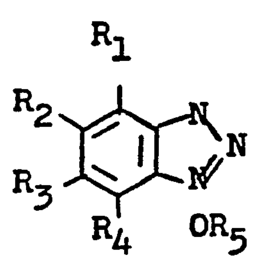

(III)

in der $R_1$ bis $R_5$ die obige Bedeutung haben, in Gegenwart eines für die Einführung des Restes $R_5$ geeigneten Schwefelsäure- oder Sulfonsäureesters oder einer geringen Menge eines üblichen Phasentransferkatalysators erhitzt oder daß man

c) ein α-Halogennitrobenzol der Formel

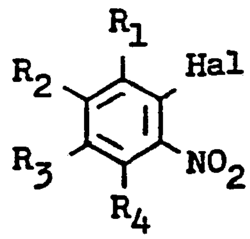

(IV)

worin $R_1$ bis $R_4$ die obige Bedeutung haben und Hal für Fluor, Chlor oder Brom steht, in einem inerten Lösungsmittel mit einem Hydrazin der Formel

$R_5$-NH-$NH_2$ (V)

in Gegenwart von basischen Substanzen umsetzt oder daß man

d) ein Benzotriazol der Formel

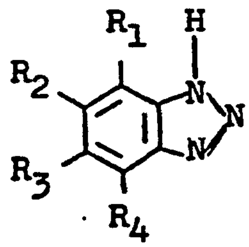

(VI)

am Stickstoff substituiert

und daß man gewünschtenfalls die nach a) bis c) erhaltenen N-Oxide nach üblichen Verfahren zu Verbindungen der Formel I mit n gleich 0 reduziert.

**Claims**

1. Compounds of formula

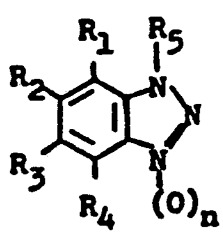

(I)

wherein

n represents 0 or 1,

$R_1$, $R_2$, $R_3$ and $R_4$, represent H, F, chlorine, bromine, $CF_3$, CN, $C_{1-4}$ alkyl, OR or SR (wherein R represents optionally oxygen-interrupted and/or chlorine-substituted $C_{1-6}$ alkyl, whilst H may be present up to three times, CN, OR and SR may be present up to not more than twice $CF_3$ may be present not more than once),

$R_5$ represents a $C_{1-8}$ alkyl or $C_{3-6}$ alkenyl group, with the proviso that if n represents 1 and $R_4$ represents hydrogen the two combinations $R_1$ and $R_2$ representing chlorine and $R_3$ representing hydrogen and $R_1$, $R_2$ and $R_3$ representing chlorine are excluded.

2. Compounds as claimed in claim 1, wherein n represents 0.

3. Compounds as claimed in claim 1, wherein n represents 1.

4. Compounds as claimed in claim 1, 2 or 3, wherein $R_5$ is methyl.

5. 3-Methyl-4-chloro-benzotriazole.

6. 3-Methyl-4,5-dichloro-benzotriazole.

7. 3-Isopropyl-4,5-dichloro-benzotriazole.

8. 3-Methyl-4-chloro-5-fluoro-benzotriazole-N-oxide.

9. 3-Methyl-5-fluoro-6-chloro-benzotriazole-N-oxide.

10. 3-Methyl-4,5,6,7-tetrachloro-benzotriazole-N-oxide.

11. 3-Methyl-4-chloro-benzotriazole-N-oxide.

12. Biocidal agent, particularly for combating phytopathogenic fungi, characterised in that it contains a compound as claimed in claims 1 to 11.

13. Use of compounds as claimed in claims 1 to 11 for combating phytopathogenic fungi.

14. Process for preparing compounds as claimed in claims 1 to 11, characterised in that

a) a benzotriazole of formula

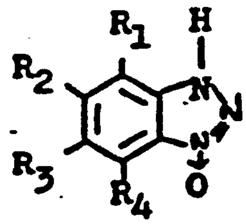

(IIa)

wherein $R_1$ to $R_4$ are defined as hereinbefore, is reacted with a sulphuric or sulphonic acid ester suitable for introducing the group $R_5$ or with a bromide $R_5$ Br, whilst if the reaction is carried out with $R_5$ Br heating is carried out in the presence of a catalytic quantity of a suitable sulphuric or sulphonic acid ester or optionally in the presence of a conventional phase transfer catalyst and, if desired, the resulting compounds of formula I wherein n = 1 are converted, in a manner known per se, by reaction with phosphorus trichloride into compounds of formula I wherein n = 0 or

b) a compound of formula

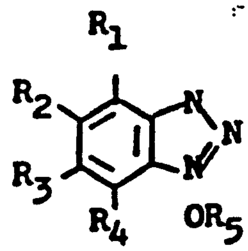

(III)

wherein $R_1$ to $R_5$ are defined as hereinbefore, is heated in the presence of a sulphuric or sulphonic acid ester suitable for introducing the group $R_5$ or in the presence of a small quantity of a conventional phase transfer catalyst or

c) an α-halonitrobenzene of formula

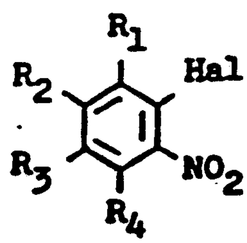

(IV)

wherein $R_1$ to $R_4$ are defined as hereinbefore and Hal represents fluorine, chlorine or bromine, is reacted in an inert solvent with a hydrazine of formula

$R_5$-NH-$NH_2$ (V)

in the presence of basic substances or

d) a benzotriazole of formula

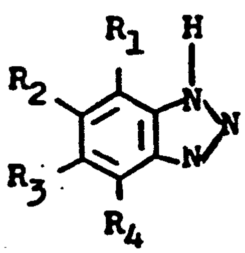

(VI)

is substituted at the nitrogen

and, if desired, the N-oxides obtained according to a) to c) are reduced by conventional methods to yield compounds of formula I wherein n equals 0.

**Revendications**

Composés de formule

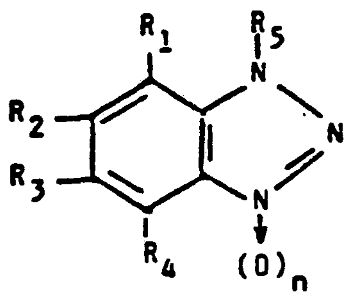

(I),

dans laquelle
n représente 0 ou 1,

$R_1$, $R_2$, $R_3$ et $R_4$ représentent H, F, chlore, brome, $CF_3$, CN, alcoyle en $C_1$-$C_4$, OR ou SR (R étant un radical alcoyle en $C_1$-$C_6$ éventuellement interrompu par oxygène et/ou substitué par chlore), H pouvant être présent jusqu'à trois fois, CN, OR et SR au maximum deux fois, $CF_3$ au maximum une fois,

$R_5$ représente un radical alcoyle en $C_1$-$C_8$ ou un radical alcényle en $C_3$-$C_6$,

étant entendu que, dans le cas où n représente 1 et $R_4$ représente l'hydrogène, les deux combinaisons de significations $R_1$ et $R_2$ chlore, $R_3$ hydrogène ainsi que $R_1$, $R_2$ et $R_3$ chlore sont exclues.

2. Composés selon la revendication 1, dans lesquels n est 0.
3. Composés selon la revendication 1, dans lesquels n est 1.
4. Composés selon la revendication 1, 2 ou 3, dans lesquels $R_5$ est méthyle.
5. Le 3-méthyl-4-chlorobenzotriazole.
6. Le 3-méthyl-4,5-dichlorobenzotriazole.
7. Le 3-isopropyl-4,5-dichlorobenzotriazole.
8. Le 3-méthyl-4-chloro-5-fluorobenzotriazole-N-oxyde.
9. Le 3-méthyl-5-fluoro-6-chlorobenzotriazole-N-oxyde.
10. Le 3-méthyl-4,5,6,7-tétrachlorobenzotriazole-N-oxyde.
11. Le 3-méthyl-4-chlorobenzotriazole-N-oxyde.
12. Agent biocide, en particulier pour lutter contre les champignons phytopathogènes, caractérisé par une teneur en un composé selon l'une des revendications 1 à 11.
13. Utilisation des composés selon l'une des revendications 1 à 11, dans la lutte contre les champignons phytopathogènes.
14. Procédé pour la préparation des composés selon l'une des revendications 1 à 11, caractérisé en ce que

a) on fait réagir un benzotriazole de formule

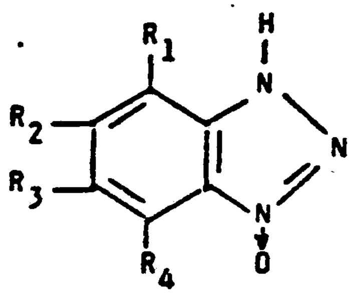

(IIa),

où $R_1$ à $R_4$ ont la signification ci-dessus, avec un ester l'acide sulfurique ou d'acide sulfonique approprié à l'introduction du radical $R_5$ ou avec un bromure $R_5Br$, selon lequel, dans le cas de la réaction avec $R_5Br$, on chauffe en présence d'une quantité catalytique d'un ester d'acide sulfurique ou d'un ester d'acide sulfonique approprié ou éventuellement en présence d'un catalyseur de transfert de phases usuel, et si on le désire on transforme les composés ainsi obtenus de formule I avec n = 1, de manière en soi connue, par réaction avec le trichlorure de phosphore, en composés de formule I avec n = 0, ou en ce que

b) on chauffe un composé de formule

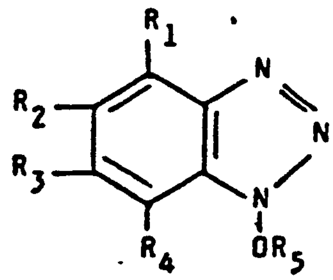

(III),

dans laquelle $R_1$ à $R_5$ ont la signification ci-dessus, en présence d'un ester d'acide sulfurique ou d'acide sulfonique approprié à l'introduction du radical $R_5$, ou d'une faible quantité d'un catalyseur de transfert de phases usuel, ou en ce que

c) on fait réagir un α-halogénonitrobenzène de formule

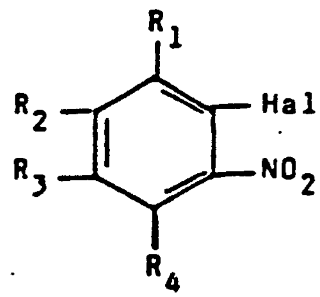

(IV),

où $R_1$ à $R_4$ ont la signification ci-dessus et Hal remplace fluor, chlore ou brome, dans un solvant inerte avec une hydrazine de formule

$R_5$ - NH - $NH_2$ (V),

en présence de substances basiques, ou en ce que

d) on substitue sur l'azote un benzotriazole de formule

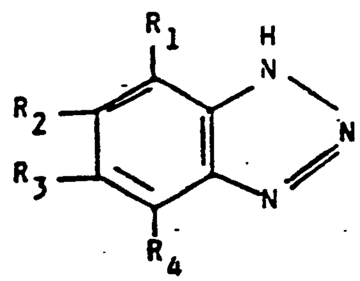

(VI),

et en ce que, si on le désire, on réduit les N-oxydes obtenus selon a) à c) selon des procédés usuels en les composés de formule I avec n représentant 0.